# EUROPEAN PATENT SPECIFICATION

(11) **EP 1 377 284 B1**
(45) Date of publication and mention of the grant of the patent: **17.12.2008**
(21) Application number: 01950733.4
(22) Date of filing: 28.06.2001
(51) Int. Cl.: A61K 31/19, A61K 31/205, A61K 9/22, A61K 9/28

(54) **CONTROLLED RELEASE ARGININE FORMULATIONS**
ZUSAMMENSETZUNGEN VON ARGININ MIT KONTROLLIERTER FREIGABE
FORMULATIONS D'ARGININE LIBERATION LENTE

(30) Priority: 28.06.2000 US 605599
(43) Date of publication of application: 07.01.2004
(73) Proprietor: Palmetto Pharmaceuticals, LLC, Lexington, MA 02420 (US)
(72) Inventor: KAESEMEYER, Wayne, H., Augusta, GA 30901 (US)
(74) Representative: Ouzman, Beverley Nicola Claire
(86) International application number: PCT/US2001/020887
(87) International publication number: WO 2002/000212

(56) References cited:
- EP-A- 0 350 246
- WO-A-00/56328
- WO-A-01/35953
- WO-A-90/11070
- WO-A-98/44893
- US-A- 5 824 331

## Description

### BACKGROUND OF THE INVENTION

A family of enzymes generically referred to as Nitric Oxide Synthase ("NOS") is responsible for forming nitric oxide from L-arginine. The nitric oxide produced is at least partially responsible for the endothelium dependent relaxation and activation of soluble guanylate cyclase, neurotransmission in the central and peripheral nervous systems, and activated macrophage cytotoxicity.

Nitric Oxide Synthase occurs in many distinct isoforms which include a constitutive form (cNOS) and an inducible form (iNOS). The constitutive form is present in normal endothelial cells, neurons and some other tissues. Formation of nitric oxide by the constitutive form in endothelial cells is thought to play an important role in normal blood pressure regulation, prevention of endothelial dysfunction such as hyperlipodemia, arteriosclerosis, thrombosis, and restenosis. The inducible form of nitric oxide synthase has been found to be present in activated macrophages and is induced in vascular smooth muscle cells, for example, by various cytokines and/or microbial products.

Although it was initially described in endothelium, NOS activity has now been described in many cell types. Brain, endothelium, and macrophage isoforms appear to be products of a variety of genes that have approximately 50% amino acid identity. NOS in brain and in endothelium have very similar properties, the major differences being that brain NOS is cytosolic and the endothelial enzyme is mainly a membrane-associated protein.

Sustained release products are widely recognized in the art and are of extreme importance in the pharmaceutical field. Through the use of such products, orally and rectally administered medications can be delivered continuously at a substantially uniform rate over a prolonged period of time so as to provide a stable, predetermined concentration of a drug in the bloodstream, without requiring close monitoring and frequent re-administration.

Sustained release is achieved by a variety of methods. Two common methods are:
1) providing a sustained release coating upon tablets or microspheres wherein slow release of the active occurs via either gradual permeation through, or gradual breakdown of, this coating; or
2) providing a sustained release matrix, such as a fat, a wax, or a polymeric material intermixed with the active ingredient in the tablet itself. These are described for example in "Sustained Action Dosage Forms" The Theory and Practice of Industrial Pharmacy, Manford Robinson ch. 14 (L. Lachman et al., eds., 2d ed., 1976).

Sustained release matrix formulations are typically prepared by methods involving pre-granulating the active ingredient together with the matrix material via a wet granulation, solvent granulation, shear-melt or roto-melt granulation, or a wet pre-adsorption technique. In these techniques, a liquid phase is used in order to uniformly mix and/or closely contact the ingredients together so as to provide an evenly distributed matrix in intimate association with the active ingredient. These formation processes help prevent creation of interspersed quick-release zones which would result in discontinuous dissolution of the tablet and thus cause bioconcentration spikes of active ingredient in the patient. Also, they frequently result in tablets of a relatively higher density than the dry mixed ones, thus allowing the use of tablets, for a given dose, that are smaller than those made by dry mixing for the same intended release rate.

U.S. Pat. No. 4,259,314 to Lowey employs a mixture of cellulose ethershydroxypropylmethylcellulose ("HPMC") and hydroxypropyl cellulose--to form a sustained release matrix in which the cellulose ether mixture has a weighted average viscosity rating of 250-4500 centipoise.

U.S. Pat. No. 5,451,409 to Rencher et al. discloses a dry mixed tablet in which a mixture of hydroxypropyl cellulose and hydroxyethyl cellulose forms the sustained release matrix; 0.5-10% HPMC is also added as a binder.

U.S. Pat. No. 4,369,172; U.S. Pat. No. 4,389,393, and U.S. Pat. No. 4,983,396 to Forest discuss the use of HPMC in a variety of formulations.

### SUMMARY OF THE INVENTION

The administration of L-arginine alone has been shown to restore vascular NO activity in animals and in humans with vasodilator dysfunction. The use of L-arginine or its biological equivalents alone, and in combination with a variety of NOS agonists, has been shown to have an unexpected beneficial effect. U.S. Patent No. 5,543,430; U.S. Patent No. 5, 767, 160; and U.S. Patent No. 5,968,983 discuss some of these formulations, their applications, and the benefits seen with the administration of these active ingredients. The therapeutic value of L-arginine when mixed with certain other agents is clear.

The present invention relates to L-arginine (for example and preferably L-arginine hydrochloride) formulated in a controlled release or sustained release formulation. Generally a carrier base material is combined with L-arginine, alone or in combination with another agent (e.g. nitrates, statins, etc.) which stimulates the production of Nitric Oxide. The ingredient(s) are manipulated into a solid, shaped dosage unit having a long-lasting and regular incremental release of the L-arginine or other medicant. The preferred embodiment of the present invention uses HPMC as the carrier base material. It would appear that a sustained release formulation of L-arginine either alone or in combination with another agent which enhances the biotransformation of L-arginine or a NOS agonist (e.g. nitrates or Hmg-CoA reductase inhibitors such as pravastatin) would have a heretofore unexpected benefit.

The term "subject" as used herein means any mammal, including humans, where nitric oxide ("NO") formation from arginine occurs. The methods described herein contemplate prophylactic use as well as curative use in therapy of an existing condition. The term "native NO" as used herein refers to nitric oxide that is produced through the bio-transformation of L-arginine or in the L-arginine dependent pathway. The term "endpoints" as used herein refers to clinical events encountered in the course of treating cardiovascular disease, up to and including death (mortality).

"L-arginine" as used herein is intended to includes all biochemical equivalents (*i.e.,* salts, precursors, and its basic form) of L-arginine, preferably those that act as substrates of NOS with resulting increase in production of NO. For example, L-lysine may be a biological equivalent of L-arginine. Other bio-equivalents of L-arginine may include arginase inhibitors, citrulline, ornithine, and hydralazine. As used herein a "biological equivalent" is an agent or composition, or combination thereof, which has a similar biological function or effect as the agent or composition to which it is being deemed equivalent.

"Agonist" refers to an agent which stimulates or enhances the bio-transformation of a NO precursor, such as L-arginine or L-lysine to NO either through enzymatic activation, regulation or increasing gene expression (*i.e.,* increased protein levels of c-NOS). Of course, either or both of these mechanisms may be acting independently, consecutively, or simultaneously.

In one embodiment of the present invention there is provided a method for providing a sustained release administration of L-arginine or a biological equivalent of L-arginine. The method allows a relatively constant release of arginine over a pre-determined amount of time. This is important due to what appears to be a supply-demand mismatch of L-arginine vis-a-vis NOS.

An alternative embodiment of the present invention provides a sustained release formulation comprised of L-arginine and an Hmg-CoA reductase inhibitor, preferably atorvastatin, pravastatin, or simvastatin, and more preferably pravastatin.

An alternative embodiment of the present invention provides a sustained release formulation comprised of L-arginine and an angiogenic growth factor. An alternative embodiment of the present invention provides a sustained release formulation comprised of L-arginine and DOX.

An alternative embodiment of the present invention provides a sustained release formulation comprised of an arginine based mixture, said arginine based mixture including a biological equivalent of arginine and an agent which enhances the bioavailability of nitric oxide. In a preferred embodiment of the present invention, the biological equivalent of arginine is L-arginine. The biological equivalent of arginine may be an arginase inhibitor, a nitrate, an angiogenic growth factor, DOX or an Hmg-CoA reductase inhibitor. The preferred Hmg-CoA reductase inhibitor is pravastatin.

Importantly, a slow release arginine formulation provides substantially constant release of L-arginine over a pre-determined period of time, thereby ameliorating the supply-demand mismatch involved with vasodilation or pathologies associated therewith.

### BRIEF DESCRIPTION OF THE DRAWINGS

Fig. 1A is the top portion of a schematic representation of proposed L-arginine dependent and independent pathways;
Fig. 1B is the bottom portion flowing from Fig. 1A of a schematic representation of the proposed L-arginine dependent and independent pathways;
Fig. 2 is a bar graph illustrating the stimulation of NOS with pravastatin;
Fig. 3 is a graph illustrating the dissolution of 350 mg controlled release ethylcellulose core arginine tablets over time;
Fig. 4 is a graph illustrating the dissolution of 350 mg controlled release ethylcellulose core arginine tablets having a HPMC or Surelease® over time;
Fig. 5 is a graph illustrating the dissolution of 350 mg controlled release HPMC core arginine tablets over time;
Fig. 6 is a graph illustrating the dissolution of 350 mg controlled release HPMC core arginine tablets having a HPMC or Surelease® over time; and
Fig. 7 is a graph illustrating the dissolution of 350 mg controlled release Kollidon® core arginine tablets over time.

### DESCRIPTION OF THE SPECIFIC EMBODIMENTS

The present invention provides the introduction of a therapeutic agent in a sustained or controlled release formulation which includes at least a NO precursor. More preferably the NO precursor is used in combination or in conjunction with an agent which enhances the conversion of the NO precursor to NO. Of particular interest as the NO precursor is L-arginine and its biological equivalents, especially L-arginine hydrochloride.

Depending on the intended use of the sustained release formulation, therapeutic agent(s) may be incorporated in a pill or tablet form or deposited in or coated on the body of a sustained release device (e.g. in a polymeric matrix). The sustained release formulation is preferably comprised of the NO precursor agent. The NO precursor agent in the sustained release formulation may be used with simultaneous or consecutive administration of other active agents (e.g., a NOS agonist such as nitroglycerin or an Hmg-CoA reductase inhibitor such as pravastatin). By appropriate choice of the material for the sustained release formulation, a physiologically active amount of the.NO precursor agent and/or therapeutic mixture may be maintained for an extended period of time (e.g. one day and up to a week or more) depending on the form of administration and the acceptability of this form. The amount of the NO precursor agent or therapeutic mixture has been and will be determined empirically in accordance with known techniques using animal and human models.

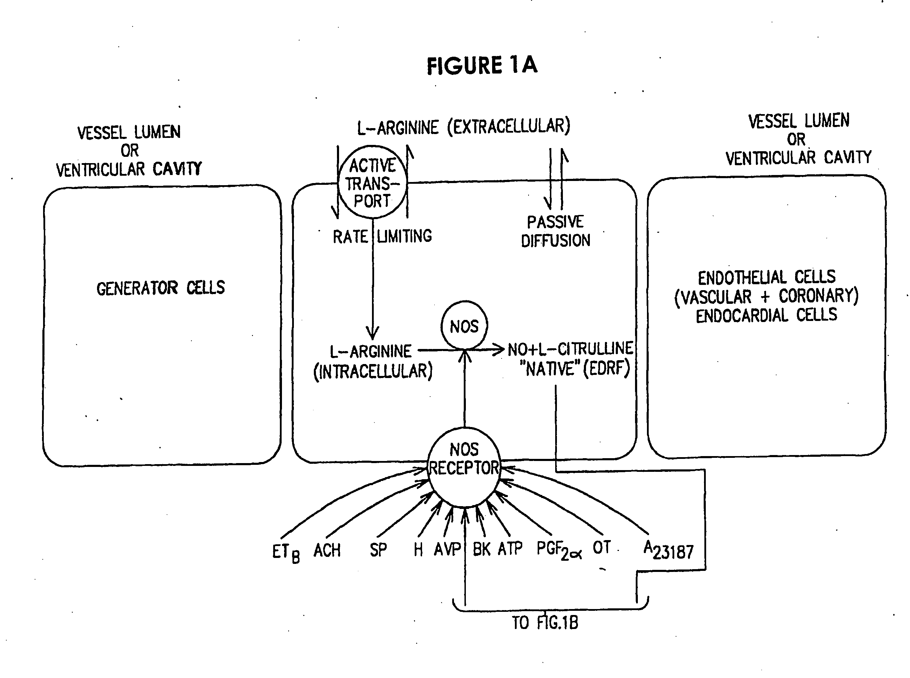

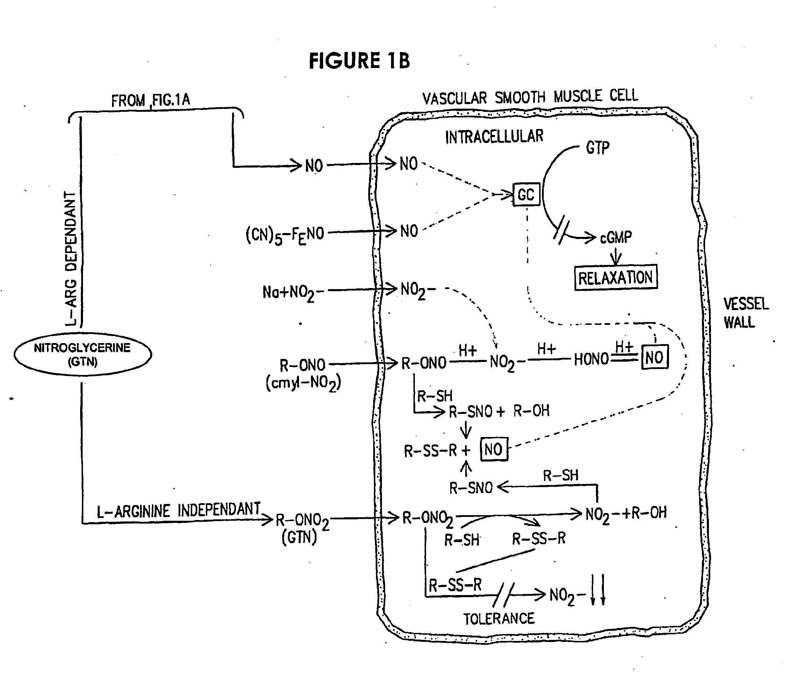

Fig. 1A and Fig. 1B illustrate a schematic representation of the proposed mechanism of action elicited by nitrovasodilators on both a generator cell and a target cell and their interrelationship. It appears that nitroglycerin or glycerol trinitrate's (GTN) mechanism of action is both L-arginine dependent and L-arginine independent and this implication has far reaching effects regarding the development and treatment of nitroglycerin tolerance and reducing clinical endpoints and mortality. Research into the area of NOS activation reveals a number of agonists of NOS some of which have been described in U.S. Pat. No. 5,543,430, U.S. Pat. No. 5,767,160, and U.S. Patent No. 5,968,983.

As shown in Figs. 1A and 1B the production of NO may result from a variety of sources and mechanisms which are discussed in detail in Ignarro, (Louis J. PhD., 1991, Pharmacology of Endothelium-Derived Nitric Oxide and Nitrovasodilators, The Western Journal of Medicine, pp.51-62.). Although this discussion focuses on smooth muscle and myocyte relaxation, cNOS, endothelial cells, and vascular smooth muscle cells, this illustration is not intended in any way to imply any cellular relationship between the various sites of action, but rather is meant to illustrate their proposed functional relationship. It is hypothesized herein and in related cases that the tolerance involves the L-arginine dependent pathway or endothelium, dependent pathway shown in Figs. 1A and 1B. As seen in Fig. 1A, the generator cell is known to have several receptor mediated agonists such as Endothelium B receptor (ET_{B}); acetylcholine (Ach); substance P (SP), Histamine (H); arginine vasopressin (AVP); bradykinin (BK); Adenosine Triphosphate (ATP); Prostaglandin F_{2α}. (F_{2α}); Oxytocin; (OT); and the calcium ionophore (A23187) which stimulate the production of NOS.

Combining L-arginine or biologically equivalents thereto with an agent which enhances its conversion enhances the action of NO dependent response. For example, sustained administration (e.g., L-arginine four times daily) overcomes or ameliorates the resistance or tolerance level normally seen when administering nitroglycerin alone. It is thought that sufficient L-arginine over a pre-determined time provides additional substrate for the stimulated nitric oxide synthase which catalyzes the biotransformation of L-arginine into nitric oxide.

As shown in Fig. 1B, under conditions leading to tolerance the agonist effect of nitroglycerin on NOS induction leads to a depletion of L-arginine in the endothelial cell. By adding L-arginine when administering nitroglycerin or when tolerance is indicated it is believed that EDRF can be generated, and in the process a significant reduction in clinical and mortality endpoints can be obtained relative to using nitroglycerin alone or in combination with SNP or other donors of exogenous NO. Clinical data supports this proposition wherein treadmill time of individuals is nitate tolerance increased when they were given four times daily administration of L-arginine as compared to placebo.

In one embodiment of the invention, therapeutically effective amounts of L-arginine and inhibitors of Hmg-CoA reductase are mixed at a physiologically acceptable pH in a sustained release formulation and administered to a patient. Of course in the sustained release formulation the L-arginine may be formulated alone or in combination with the Hmg-CoA reductase inhibitor. If L-arginine is formulated alone in a sustained release formulation, the Hmg-CoA reductase inhibitor is administered in conjunction (e.g. consecutively, simultaneously, or within release period) of the sustained release L-arginine. A preferred Hmg-CoA reductase for this purpose is pravastatin. The fact that Hmg-CoA reductase may be an agonist or stimulant of nitric oxide synthase has remarkable implications.

L-arginine may be used in conjunction with virtually any of the family of those substances known as Hmg-CoA reductase inhibitors. These are taught for example in U.S. Pat. Nos. 4,857,522,5,190,970, and 5,461,039.

Those particular Hmg-CoA reductase inhibitors most preferred for use in conjunction with the present formulation as selected from the group consisting of atorvastatin, cerivastatin, simvastatin, lovastatin, pravastatin, compactin, fluvastatin, and dalvastatin. U.S. Patent No. 5,316,765 cites a number of these Hmg-CoA reductase inhibitors In particularly preferred embodiments of the present invention, the Hmg-CoA reductase inhibitor utilized is pravastatin, simvastatin, or atorvastatin. In an even more particularly preferred embodiments, the administration of the present invention includes the Hmg-CoA reductase inhibitor pravastatin. Also within the scope of those Hmg-CoA reductase inhibitors of the present invention are included the bio-active metabolites of those Hmg-CoA reductase inhibitors described here, such as pravastatin sodium (the bio-active metabolite of mevastatin). Any one or several of the Hmg-CoA reductase inhibitor compounds may be mixed with L-arginine or substrate precursor to endogenous nitric oxide to provide a therapeutically effective mixture. This therapeutically effective mixture can then be incorporated into a sustained release formulation or other delivery device.

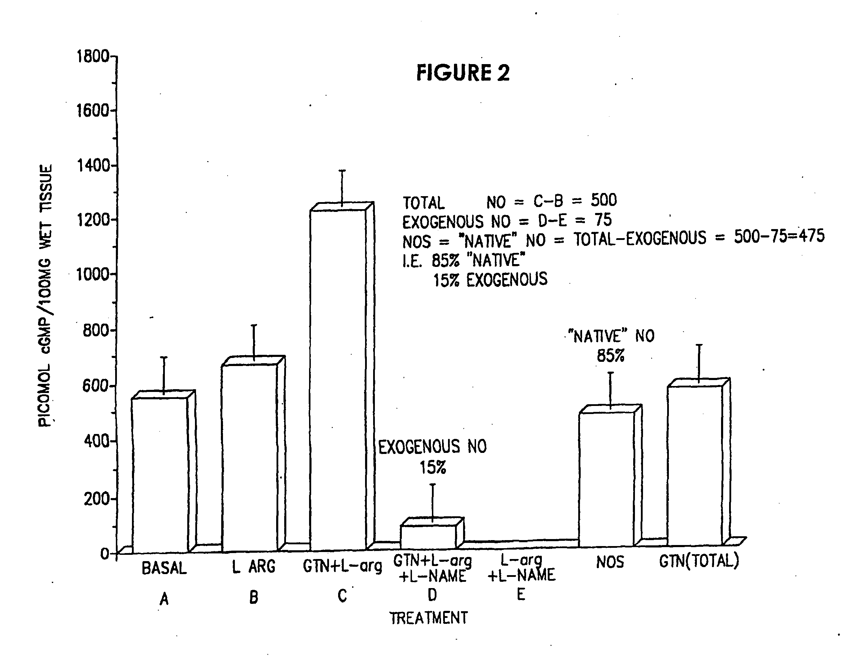

To demonstrate the levels of NO production, the direct effects of acteylcholine and pravastatin on NO production in bovine aortic endothelial cells (BAEC) was determined using a highly sensitive photometric assay for conversion of oxyhemoglobin to methemoglobin. NO oxidize; oxyhemoglobin (HbO₂) to methemoglobin (metHb) in the following reaction HbO₂, + NO - metHb + NO₃. The amount of NO produced by endothelial cells was quantified by measuring the change in absorbance as HbO₂ oxidizes to metHb. Oxyhemoglobin has an absorbance peak at 415 nm, while metHb has a 406 nm absorbance peak. By subtracting the absorbance of metHb from HbO₂, the concentration of NO can be assessed. The general method was patterned after that of Feelisch et al., (Biochem. and Biophy. Res. Comm. 1991; 180, Nc 1:286-293). Fig. 2 is a bar graph of the data generated which illustrates the effects of acetylcholine and pravastatin (10⁻⁶ and 10⁻⁵ M) administered for 3 min periods into the cell/bead perfusion system on NO production with: 1) 10⁻⁵ M L-arginine in control (basic) buffer, 2) 10⁻³ M of L-NAME in buffer, and 3) 10⁻³ M of L-arginine in buffer. Responses are transient elevations in NO production above basal levels. Data for responses in L-NAME and L-arginine augmented buffer are presented as percent of response in control buffer (100%); numbers in basic buffer bars indicate absolute production of NO in nmole *min. The remaining two bars denote differences between responses in L-NAME buffer vs both basic and L-arginine added buffers.

Many of the NOS agonists originally identified have also been implicated in angiogenesis. Substance P ("SP"), a secretory product, is identified herein as a cNOS agonist. Other secretory products (e.g., those identified in "Macrophages and angiogenesis" by Sunderkotter et al. (J Leukoc Biol 1994 Mar; 55(3):410-22)) may also be expected to be agonists of NOS. Bradykinin ("BK"), a NOS agonist, has also been implicated as a possible angiogenic factor. Angiogenic growth factors like those identified in Table I stimulate the growth of new blood vessels (e.g., in vascular beds such as the coronary, peripheral, etc.) previously occluded with atherosclerotic obstructions. Angiogenic growth factors are proteins which were initially discovered as agents responsible for the growth of new blood vessels which maintain the growth and spread of cancerous tumors (neovascularization). Two of the angiogenic growth factors, vascular endothelial growth factor (VEGF) and basic fibroblastic growth factor (bFGF) result in the growth of significant new collateral blood vessels.

Like angiogenic agents Substance P and Bradykinin, VEGF and bFGF also appear to act as NOS agonists, specifically cNOS. It appears the resultant EDNO produced is in large part responsible for the new collateral vessel growth ("collateral") which in turn is responsible for the improvement in symptoms of ischemia seen in therapeutic angiogenesis. Furthermore, it has also been shown that the collateral responses to both VEGF and bFGF can be magnified significantly with L-arginine supplementation. Therefore, angiogenic growth factors, preferably VEGF and bFGF, appear to have dual applicability in the treatment of hypertension and cardiovascular diseases in that they both stimulate therapeutic angiogenesis and activity of Nitric Oxide Synthase. It also appears that the overall therapeutic angiogenic result with angiogenic growth factors is augmented to the extent they act as agonists of NOS. The fact that angiogenic growth factors are agonists or stimulators of nitric oxide synthase has important implications. Mixing angiogenic growth factors "in vitro" or "in vivo" with L-arginine may have an unforeseen beneficial effect that is associated with excess L-arginine providing additional substrate for NOS and the NOS being catalyzed to enzymatically increase the biotransformation of L-arginine into nitric oxide (EDRF or EDNO) which would in turn amplify the overall therapeutic effect.

L-arginine may be used in conjunction with any of the family of those substances known as angiogenic growth factors. However, those particular angiogenic growth factors most preferred for use in conjunction with the present formulation are selected from the group consisting of VEGF and bFGF and even more preferably VEGF. Of course these agents may be over-expressed by administration of a particular agent in combination with a sustained release formulation of L-arginine. Although it is with particular reference to VEGF and bFGF it should be noted that genetic over-expression of a NOS agonist or other bio-active agent as described herein is specifically as contemplated in combination with the controlled or sustained release of arginine. VEGF can be obtained from Genentech (South San Francisco, CA) and bFGF can be obtained from R&D Systems (Minneapolis, MN). The range of ratios of an angiogenic growth factor to L-arginine may be employed with virtually any of the angiogenic growth factors.

Compositions of the present invention may include agents such as a stabilizing compound, which may be administered in any sterile, bio-compatible pharmaceutical carrier, including, but not limited to, saline, buffered saline, dextrose, and water. The compositions may be administered to a patient alone, or in combination with other agents, drugs or hormones. Pharmaceutically-acceptable carriers may also be comprised of excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, PA). The pharmaceutical composition may be provided as a salt and can be formed with many acids, including but not limited to, hydrochloric, sulfuric, acetic, lactic, tartaric, malic, succinic, etc.

After the controlled release compositions have been prepared, they can be placed in an appropriate container and labeled for treatment of an indicated condition. Such labeling would include amount, frequency, and method of administration.

The exact dosage of the present invention will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy.

The theory and mechanism presented herein are provided solely to further elucidate the invention. An alternative embodiment of the present invention is based on a the fact that when cellular supply of L-arginine is limited, NLOS utilizes molecular oxygen as a lone substrate producing superoxide anion and other reactive free radicals which can lead to cardiovascular dysfunction and the pathogenesis of disease. Thus a sustained release formulation of arginine would appear to be very useful in ameliorating the conditions caused by depletion of L-arginine. The total intracellular concentration of L-arginine (0.1 - 1mM) in endothelial cells (EC) greatly exceeds the Km of eNOS for L-arginine. This suggests that eNOS is saturated with substrate and that levels of intracellular L-arginine are not limiting for NO production. However, other studies have shown that availability of L-arginine varies greatly within the EC due to intracellular compartmentalization and dequestration in addition to degradation by arginase or the presence of endogenous inhibitors of eNOS (i.e., asymmetrical dimethylarginine). Recently, it has also been shown that concurrent cellular L-arginine transport may be more important than intracellular L-arginine levels in providing L-arginine to NOS for NO production. Therefore, total intracellular concentration of L-arginine may not truly reflect the L-arginine available at the site of NOS action.

Supply of L-arginine may become limiting and reduce formation of NO in normal and pathological states. Treatment of guinea pigs with L-arginine has been shown to increase the duration of the vasodilatory response to acetylcholine under normal physiological conditions; prior stress with norepinephrine infusion accentuates this enhancement process. It has been demonstrated that acetylcholine and a Ca++-ionophore which release NO can induce tolerance in isolated arterial rings. Tolerance was associated with depletion of L-arginine and was reversed with L-arginine repletion. L-arginine may also become limiting under pathologic conditions. Endothelial dysfunction in cardiomyopathic hamsters can be reversed by L-arginine. In addition, humans with acute hyperglycemia exhibit intense vasoconstriction and impaired endothelial function which can be completely reversed by intravenous infusions of low concentrations of LA. Other diseases in which pathology is attributed to a deficiency of Larginine include hypertension, atherosclerosis, restenosis - post coronary angioplasty and reperfusion injury. Similarly, addition of L-arginine in these circumstances also ameliorates the deficit in endothelium-dependent relaxation.

Intracellular L-arginine is derived from several sources including the transport of L-arginine into cells, amount of intracellular L-citrulline recycled back to LA, rate of degradation of L-arginine (arginase), incorporation of L-arginine into proteins (compartmentalization) and the amount of L-arginine utilized upon activation of intracellular NOS. Uptake of L-arginine into EC occurs through two carrier-mediated transporters and passive diffusion. The saturable carrier-mediated transporters include a sodium-dependent active transporter, system B + and a sodium-dependent transporter, system y⁺. The majority (80%) of L-arginine delivered into most cells is through the y⁺ transporter. Regulation of L-arginine transport appears to involve cellular membrane potential.

When the balance of transporter regulatory factors is negative, L-arginine supply becomes limiting and subsequent production of O₂ may contribute to vascular and organ pathology. We compared the effects of NOS agonists and NO donors on L-arginine uptake by EC. Effects of NOS stimulation on superoxide anion production were also assessed in the presence and absence of L-arginine and the NOS antagonist, L-NAME.

It appears L-arginine levels are maintained primarily through the activity of the carrier-mediated Na⁺-independent transporter, y⁺, while the Na⁺-dependent transporter, B^{α+}, and passive diffusion account for less than 15%. Concurrent transport of L-arginine to NOS may control NO production. However, L-arginine supply to NOS can be limiting due to compartmentalization within EC, arginase activity or utilization of L-arginine by NOS. We believe that NO and superoxide anion both appear to reduce the activity of the y⁺ transporter and also reduce L-arginine available for NOS. Collectively, summation of supply verses demand or availability of L-arginine to NOS will determine whether NO or superoxide anion are formed.

Interestingly, data for the NO donor, SNAP, depicts initial stimulation of the y⁺ transporter within 10 minutes followed by no change and then inhibition of cellular L-arginine uptake with more prolonged exposures to NO, a "cross-over" effect. An initial increase of cellular uptake of L-arginine is expected as NO is known to cause cellular hyperpolarization. However, longer exposures of 1 to 4 hours resulted in a marked reduction of L-arginine transport. These data were confirmed by using a different NO donor, DPTA, to stimulate prolonged exposure of cells to NO. DPTA releases NO slowly over time and, therefore, was used to repeat the longer durations of NO exposure.

It appears that concurrent L-arginine supply to NOS via system y⁺, independent of overall intracellular L-arginine, is critical in establishing and maintaining vascular function. Factors including NOS agonists and NO itself appear to control y⁺ activity and the summation of these factors is critical in determining NO and superoxide anion formation, both of which contribute to vascular dysfunction and disease.

Due to the apparent mismatch of available arginine a sustained release formulation of arginine (or a biological equivalent thereof) to be incorporated in a tablet, capsule, or other administration route would be advantageous. Arginine in a controlled release formulation in and of itself is an improvement over the state of the art in that it supplies a relatively constant amount of arginine and overcomes the large spildng present in instant release formulations. The supply-demand mismatch heightens the need for a slow or controlled L-arginine formulation.

The preferred embodiment of the present invention comprises sustained release tablets of an active ingredient which include a sustained release HPMC or ethylycellulose matrix. In a preferred embodiment of the present invention a combination comprising at least one active ingredient together with hydroxypropylmethylcellulose (HPMC) is mixed and is directly compressed to form tablets. Preferably, the composition is prepared by dry mixing the ingredients. Preferably, one of the active ingredients is arginine or a pharmacologically acceptable salt thereof, such as arginine hydrochloride or arginine sulfate, or a mixture thereof. More preferred as an active ingredient is L-arginine hydrochloride. Preferably about 15-50% of the active ingredient, based on the final weight of the tablets, is used; more preferably, about 20-50%; most preferably about 40-45%. In a preferred embodiment, the amount of active ingredient used is that which is sufficient to produce tablets, each comprising in the range of about 100mg to about 2g active ingredient, even more preferably about 100 mg to about 1 g, even more preferably about 200 mg to about 500mg and most preferably about 350mg. In an alternate embodiment, the amount of active utilized is sufficient to produce tablets comprising about 750mg of active ingredient each. A preferred HPMC is Methocel® K100M (produced by The Dow Chemical Co. of Midland, Mich.). Preferably about 20-40% HPMC is used, more preferably about 25-30% and most preferably about 28-29% HPMC.

Glidants, fillers, and other excipients that may be used in the preferred embodiments include those described, e.g., in Handbook of Pharmaceutical Excipients (J. C. Boylan et al., eds., 1986) and in H. A. Lieberman et al., Pharmaceutical Dosage Forms: Tablets (2d ed. 1990). Excipients generally may include: binders and adhesives; disintegrants, absorbents, and adsorbents; glidants and lubricants; fillers and diluents; and colorants, sweeteners, and flavoring agents. Preferred fillers include calcium salts and sugars, for example, calcium phosphates, calcium sulfates, mannitol, lactose, and mixtures thereof. More preferred fillers include dicalcium phosphate, tribasic calcium phosphate, directly compressible calcium sulfate, directly compressible mannitol, anhydrous lactose, flowable lactose (e.g., Fast Flo® lactose produced by Foremost Farms USA of Baraboo, Wis.), and mixtures thereof. Most preferred is dicalcium phosphate (Ca₂HPO). Preferably, about 20-40% by weight filler, based on the final weight of the tablets, is employed. However, where the filler consists of one or more sugars alone, preferably about 20-30% of filler is used.

Preferred glidants include colloidal silica and precipitated silica. A preferred colloidal silica is Cab-o-Sil® produced by the Cabot Corp. of Boston, Mass.; a preferred precipitated silica is Syloid® produced by W.R. Grace Co. of New York, N.Y. Preferably, about 0.2-2% by weight of glidant, based on the final weight of the tablets, is employed. Where colloidal silica alone is used, the tablets will preferably comprise about 0.2-0.8% by weight glidant, more preferably about 0.25-0.75%. Preferred lubricants include sodium lauryl sulfate, sodium stearyl fumarate, and metal stearates, alone or in combination with stearic acid. More preferred lubricants include magnesium stearate, zinc stearate, calcium stearate, and mixtures thereof, alone or in combination with stearic acid. Preferably about 0.2-2%, by final weight of the tablets, of lubricant is used, more preferably about 0.25-1.25%. For example, where magnesium stearate is the sole lubricant, the tablets preferably comprise about 0.3-0.5% lubricant; where a magnesium stearate-stearic acid mixture is used as the lubricant, about 0.25% magnesium stearate may be mixed with as much as about 1 % stearic acid.

In the preferred embodiment mixing procedure, the active ingredient, e.g., arginine, sustained release polymer (e.g. HPMC, ethyl cellulose, Kollidon), and the filler, e.g., dicalcium phosphate dihydrate, are passed through a screen into a clean and dry blender, preferably in the order indicated. After mixing for 5 minutes, to the above mixture are added glidants, e.g. colloidal silicate, and this is then passed through a fine mesh screen and into a clean and dry blender. They are mixed for 5-20 minutes, following which a lubricant, e.g., magnesium stearate is screened into the blender and mixed in for an additional 5-15 minutes.

After the foregoing combination has been produced with thorough mixing, it is directly compressed to form tablets, i.e. any solid form, e.g., caplets. These are then coated with a pharmaceutically acceptable coating. Preferred coatings include cellulose ether-based coatings, such as HPMC-based coatings. A preferred coating is Opadry, produced by Colorcon, Inc. of West Point, Pa. Preferably about 0.54% by weight of coating is used (in terms of weight added to the uncoated tablet), more preferably about 1-2%. A wax, e.g., an edible wax such as carnauba wax may also be applied as a second coating thereover.

Numerous advantages result from the ability to use L-arginine in a sustained release dosage form. These include the use of smaller tablets which are more economical and are easy to administer. The cellulose ethers such as the hydroxypropylmethylcelluloses of the present invention are hydrophilic and tend to absorb moisture from the atmosphere. This is particularly important when the L-arginine form being used is moisture sensitive (or in the combination formulation when the agent or NOS agonist is moisture sensitive). When mixed with the active agent(s) (a biological equivalent of L-arginine alone or in combination with another agent), the mixture has excellent compressibility and the tablets prepared therefrom are hard and dense, have low friability and provide sustained release over an extended period.

The sustained release drug forms of the present invention are stable and the release rate does not change over an extended storage period. The therapeutic compositions of the present invention, in most cases, give a steady, reproducible release of the active medicament. The L-arginine compositions of the present invention can be formulated to act locally in the mouth or systemically. The L-arginine containing composition can be administered orally to transmit the active ingredients into the gastrointestinal tract and into the blood, fluids and tissues of the body without excessive peak concentrations occurring. Alternatively, the active ingredients can be formulated to act through the buccal tissues of the mouth to transmit the active ingredient directly into the blood stream thus avoiding first pass liver metabolism and by-passing the gastric and intestinal fluids which have an adverse inactivating or destructive action on many active ingredients unless they are especially protected against such fluids as by means of an enteric coating or the like. The active ingredient can also be of a type of medication which can be transmitted into the blood circulation through the rectal tissues. It is to be understood that the present invention is directed generally to an L-arginine (or biological equivalent) either alone or in combination in a sustained release formulation and thus is applicable to sublingual lozenges, suppositories and compressed tablets, the latter intended to be swallowed in unit dosage form and which upon ingestion according to a prescribed regimen give slow and regular release L-arginine.

In making up tablets containing an orally administrable systemically absorbable active component such as one of the heretofore mentioned, the oral carrier material is thoroughly intermixed with the L-arginine and other active ingredients which is also in powdered or granular form or in solution, and any other needed ingredients which are conventional in tablet making such as magnesium stearate, lactose, starch and, in general, binders, fillers, disintegrating agents and the like. The complete mixture, in an amount sufficient to make a uniform batch of tablets, e.g. 50,000, of which each contains an effective amount of active medicament, is then subjected to tableting in conventional tableting machines at compression pressures of 2000 to 16000 lbs/sq.in. and, because of the use of the specific carrier material of this invention in the production of the tablets, a product is obtained which has the desired hardness, low level of friability and a predetermined prolonged action and a regular delayed release pattern so that the medicament is available over a period of 1 to 36 hours, depending on the precise tablet size, hardness and the particular carrier composition. In this way, it is possible to produce sustained or slow continuous release tablets in a relatively simple and economical manner on a commercial scale as contrasted with the more elaborate and more complex materials and procedures heretofore employed or proposed.

The release pattern of active medicament from the carrier of the present invention can be controlled according to the particular medication and its intended therapeutic effect. For a sublingual lozenge or tablet, the release pattern may be varied from about 15 minutes to 4 hours. For orally administered tablets, the rate of release may be 2-4 hours, 4-8 hours, 8-10 hours, 10-12 hours, 12-15 hours, 15-18 hours, 20-24 hours, etc., as desired. For vaginal and rectal suppositories, the release pattern ranges from 2 to 36 hours, and can be less where indicated. Predetermined release patterns of unusually reliable and constant characteristics can be secured. This is often very important medically, especially when treating patients having coronary diseases, such as angina pectoris with nitroglycerin, or related problems of circulatory disorders or abnormal blood pressure.

A number of controlled release prototypes were formulated to determine the most suitable for a controlled release arginine tablet or capsule. The excipient used to control the release of the active ingredient (e.g., L-arginine; its biological equivalent; or a combination of either or both of these with a NOS agonist) can be a variety of excipients commonly used in control release formulation. The two most common control release excipients are hydroxylproylmethylcellulose ("HPMC") and ethylcellulose . Preferably the tablets formed with these excipients are processed by direct compression, and even more preferably are coated with a control release film. The control release film slows the initial burst of active ingredient. The following illustrative examples are provided for a better understanding of the present invention.

A typical formulation for the ethylcellulose base controlled release formulation is:

| Ingredient | % by weight of Composition |
|---|---|
| L-Arginine | 40-50% (e.g. 43.7%) |
| Ethylcellulose (e.g. Ethocel® 7 FP, Dow) | 25-30% |
| DiCalcium Phosphate, Dihyrate | 22-27% |
| Talc | 1% |
| Magnesium Stearate | 1% |
| Fumed Silica | 1% |

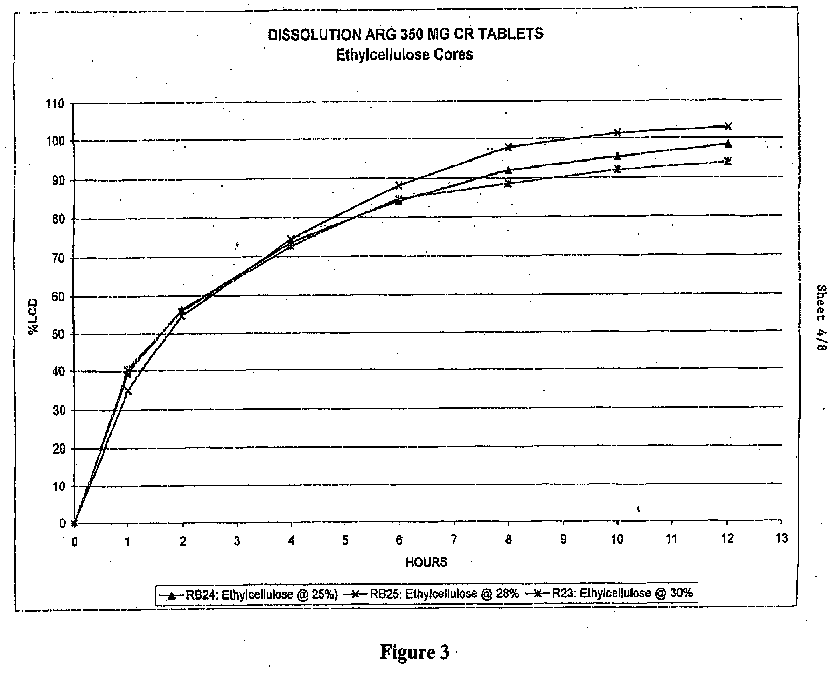

The ethylcellulose formulations demonstrate suitable, but less than ideal, flow and tablet weight variation. In an effort to ameliorate this glident levels and glident blending times were increased and ethylcellulose levels decreased. The dissolution data for Lots RB23 (30% ethylcellulose), RB24 (25% ethylcellulose) and RB25 (28% ethylcellulose) are shown in Figure 3. Arginine dissolution from these formulations is similar to the HPMC formulations.

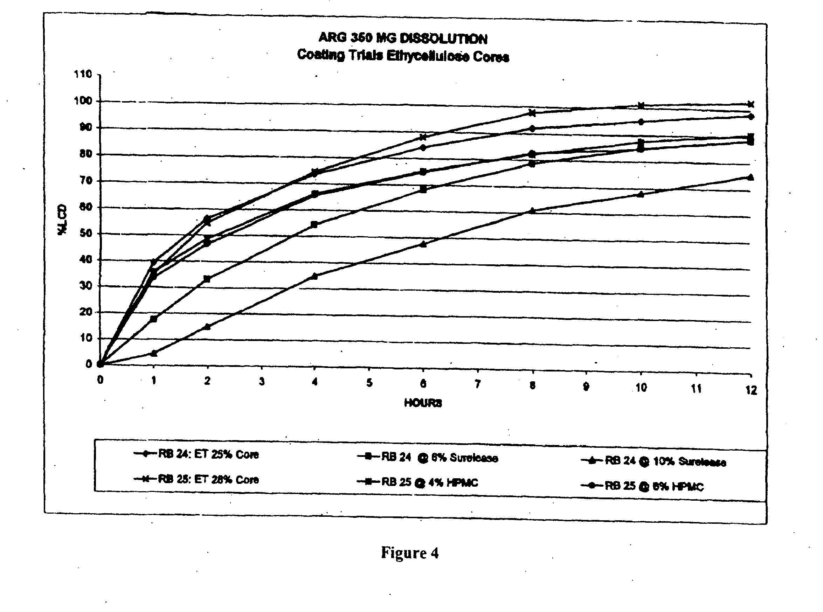

Coating trials on the ethylcellulose tablets were also conducted. As discussed above, these coatings are designed to slow down arginine release from the tablets. As can be seen in Figure 4, HPMC and modified Surelease® tablet coating formulations were evaluated. The Surelease® coating had the desired effect and slowed arginine release of the 25% ET formulation (RB24) to a desirable profile similar to RB7. The HPMC coating levels tested, 4% and 6%, did little to slow down the initial arginine release. Higher HPMC coating levels therefore appear to be more suitable and a coating level of 10% HPMC would appear to be suitable.

A typical formulation for the Hydroxypropymethyl cellulose ("HPMC") based controlled release formulation is:

| Ingredient | % by weight of Composition |
|---|---|
| L-Arginine | 40-50% (e.g. 43.7%) |
| HPMC | 28-30% |
| DiCalcium Phosphate, Dihyrate | 25-27% |
| Talc | 1% |
| Magnesium Stearate | 1% |
| Fumed Silica | 5% |

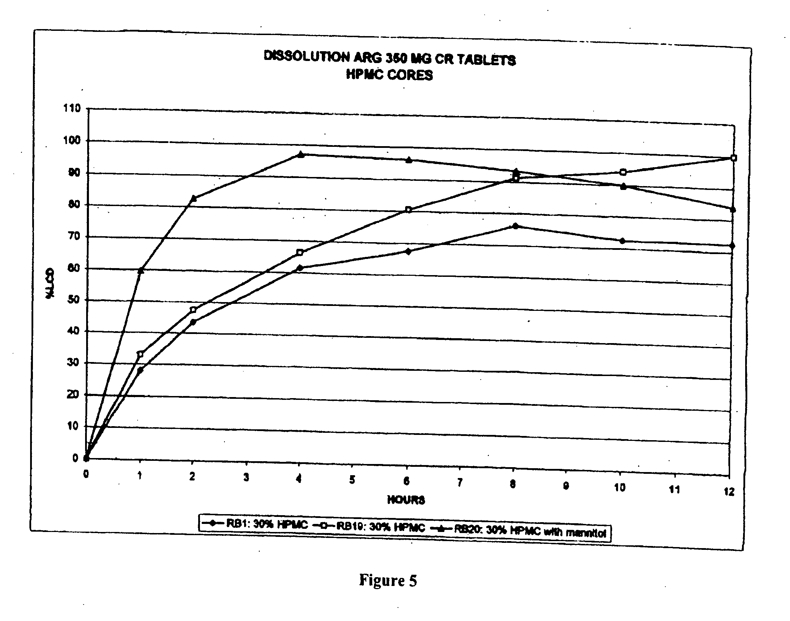

The HPMC formulations have shown an extended arginine release profile. Figure 5 compares the reproducibility of the 30% HPMC (Lots RB 1 & RB 19) using the water insoluble dicalcium phosphate tablet binder. The two profiles are substantially the same. Lot RB20 shows the effect of changing to the water soluble tablet binder mannitol. This change dramatically speeds up Arginine dissolution from the tablet. Mannitol was selected, as it is a preferred diluent for a combination product (especially for the NOS agonist is IsoSorbide Mononitrate). The amount of mannitol used in Lot RB20 exceeds the amount contemplated for use in the combination product. Therefore, the release profile should not be as fast as shown in Figure 5.

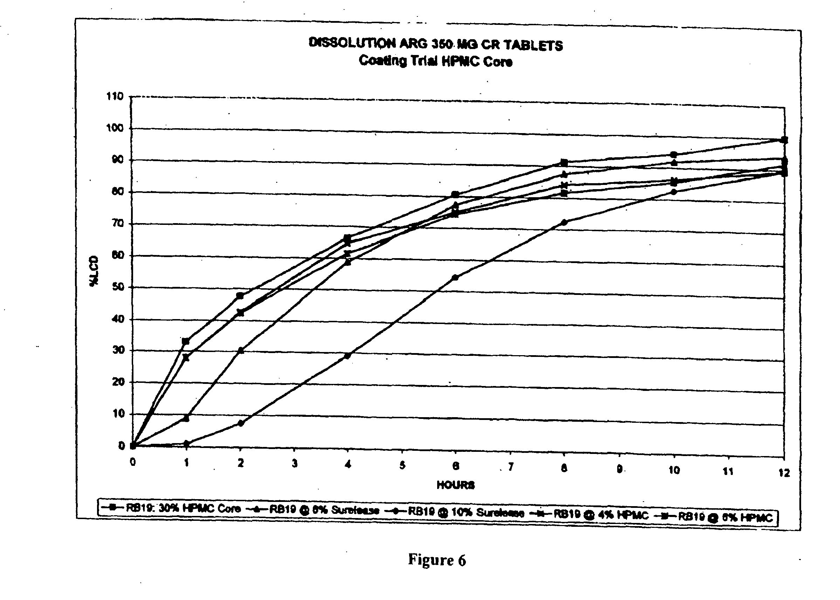

Coating trials for the HPMC tablets were also conducted. As before, these coatings are designed to slow down arginine release from the tablets. As can be seen in Figure 6, HPMC and modified Surelease® tablet coating formulations were evaluated. The Surelease® coating slowed the initial arginine release. The 10% coating level delayed the onset of release while the 6% level significantly slowed the release in the first hour. Accordingly, it appears higher percentage HPMC levels would be more suitable.

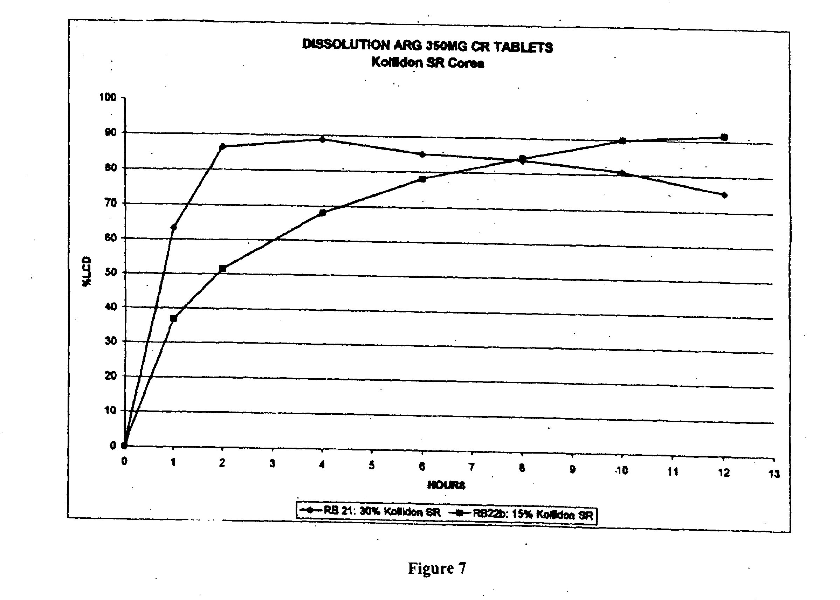

Kollidon®, a relatively new controlled release excipient, was also tested. A 30% (RB21) and a 15% formulation (RB22b) were evaluated. Interestingly the 15% concentration had a slower dissolution profile than the 30%, see Figure 7. The 15% formula has a profile that is similar to the uncoated 30% HPMC formulation. It would appear that coating samples of the 15% formulation with Surelease® at the 6-10% range would have a similar effect of slowing down arginine release as it does for the HPMC core. It should be noted that the 30% formulation processed exceptionally well while for the 15% formulation it was difficult to obtain desirable tablet hardness. The tablet can be substantially reduced by using a sustained release formulation. This is due to the fact that L-arginine has a relatively fast half life in the bloodstream. Accordingly, a controlled release formulation of 350 mg may have the overall therapeutic impact of much larger doses (e.g. 1g). Accordingly a feasible tablet for ingestion can be manufactured. When one includes the agent which enhances the biotransformation of Larginine (e.g. Imdar®) in a dose of 50 mg (assuming 80% active) with filler, and 350 mgs Larginine the tablet can be formulated as an 400mg to about 1 gram size sustained release tablet.

It may be beneficial to combine the HPMC with an alkali earth metal to slow the drug release from the tablet (e.g. sodium carbonate or any alkali metal salt of a carboxylic acid).

## Claims

1. A method for producing sustained release tablets comprising the steps of:
- mixing L-arginine with a sustained release matrix;
- compressing said mixture to form tablets; and
- coating said tablets with a coating containing at least 10% by weight hydroxypropylmethylcellulose.

2. The method of Claim 1, wherein said L-arginine is selected form the group consisting of L-arginine hydrochloride, pharmacologically acceptable arginine salts, and mixtures thereof.

3. The method as claimed in either one of Claims 1 and 2 wherein said arginine comprises 15% to 60% by weight of the tablet.

4. The method as claimed in any preceding Claim, wherein said arginine is present in an amount sufficient to produce tablets in a range from 150 mg to 2000 mg of said L-arginine.

5. The method as claimed in any preceding Claim, wherein said arginine is present in an amount sufficient to produce tablets with 750 mg of L-arginine.

6. The method as claimed in any preceding Claim, wherein said arginine is present in an amount sufficient to produce tablets with 350 mg L-arginine.

7. The method as claimed in any preceding Claim, wherein said L-arginine and said sustained release matrix are dry mixed with a glidant and a filler.

8. The method of Claim 7, wherein said glidant is selected from the group consisting of colloidal silica, precipitated silica, and mixtures thereof.

9. The method as claimed in any preceding Claim, wherein said sustained release matrix is hydroxypropylmethylcellulose (HPMC).

10. The method as claimed in any preceding Claim, wherein said tablet is coated with a coating, said coating being a cellulose ether-based coating alone or in combination with ethyl cellulose.

11. The method as claimed in any preceding Claim, further including the steps of mixing in an agent which enhances the bio-transformation of L-arginine into Nitric Oxide.

12. The method of Claim 11, wherein said agent is selected from the group consisting of a NOS agonist, an HMG-CoA reductase inhibitor, and an ACE inhibitor.

13. A composition comprised of a therapeutically effective amount of arginine; a therapeutically effective amount of a HMG-CoA reductase inhibitor other than simvastatin; and sustained release polymeric matrix.

14. The composition of Claim 13, further including a nitrate.

15. A sustained release pharmaceutical tablet comprised of a sustained release matrix; a therapeutically effective amount of arginine, and an HMG-CoA reductase inhibitor other than simvastatin.

16. The tablet of Claim 15 wherein said arginine is 20% to 60% by weight of said tablet.

17. The tablet of either one of Claims 15 and 16, wherein said arginine is selected from the group consisting of L-arginine, L-arginine hydrochloride, pharmacologically acceptable arginine salts, and mixtures thereof.

## Patentansprüche

1. Ein Verfahren zum Herstellen von Retardtabletten, das die folgenden Schritte beinhaltet:
- Mischen von L-Arginin mit einer Retardmatrix;
- Komprimieren der Mischung, um Tabletten zu bilden; und
- Beschichten der Tabletten mit einer Beschichtung, die mindestens 10 Gewichtsprozent Hydroxypropylmethylzellulose enthält.

2. Verfahren gemäß Anspruch 1, wobei das L-Arginin aus der Gruppe, die aus L-Arginin-Hydrochlorid, pharmakologisch zulässigen Argininsalzen und Mischungen daraus besteht, ausgewählt wird.

3. Verfahren gemäß einem der Ansprüche 1 und 2, wobei das Arginin 15 bis 60 Gewichtsprozent der Tablette beinhaltet.

4. Verfahren gemäß einem der vorhergehenden Ansprüche, wobei das Arginin in einer Menge vorliegt, die ausreicht, um Tabletten mit L-Arginin in einem Bereich von 150 mg bis 2000 mg herzustellen.

5. Verfahren gemäß einem der vorhergehenden Ansprüche, wobei das Arginin in einer Menge vorliegt, die ausreicht, um Tabletten mit 750 mg L-Arginin herzustellen.

6. Verfahren gemäß einem der vorhergehenden Ansprüche, wobei das Arginin in einer Menge vorliegt, die ausreicht, um Tabletten mit 350 mg L-Arginin herzustellen.

7. Verfahren gemäß einem der vorhergehenden Ansprüche, wobei das L-Arginin und die Retardmatrix mit einem Flussregulierungsmittel und einem Füllstoff trocken gemischt werden.

8. Verfahren gemäß Anspruch 7, wobei das Flussregulierungsmittel aus der Gruppe, die aus kolloidaler Kieselsäure, gefällter Kieselsäure und Mischungen daraus besteht, ausgewählt wird.

9. Verfahren gemäß einem der vorhergehenden Ansprüche, wobei die Retardmatrix Hydroxypropylmethylzellulose (HPMC) ist.

10. Verfahren gemäß einem der vorhergehenden Ansprüche, wobei die Tablette mit einer Beschichtung beschichtet ist, wobei die Beschichtung eine Beschichtung auf der Basis von Zelluloseäther allein oder in Kombination mit Ethylzellulose ist.

11. Verfahren gemäß einem der vorhergehenden Ansprüche, das ferner die Schritte des Zumischens eines Wirkstoffs, der die biologische Umwandlung von L-Arginin zu Stickstoffoxid verbessert, umfasst.

12. Verfahren gemäß Anspruch 11, wobei der Wirkstoff aus der Gruppe, die aus einem NOS-Agonisten, einem HMG-CoA-Reduktase-Hemmer und einem ACE-Hemmer besteht, ausgewählt wird.

13. Eine Zusammensetzung, die eine therapeutisch wirksame Menge an Arginin, eine therapeutisch wirksame Menge eines anderen HMG-CoA-Reduktase-Hemmers als Simvastatin und eine polymere Retardmatrix beinhaltet.

14. Zusammensetzung gemäß Anspruch 13, die ferner ein Nitrat umfasst.

15. Eine pharmazeutische Retardtablette, die eine Retardmatrix, eine therapeutisch wirksame Menge an Arginin und einen anderen HMG-CoA-Reduktase-Hemmer als Simvastatin beinhaltet.

16. Tablette gemäß Anspruch 15, wobei das Arginin 20 bis 60 Gewichtsprozent der Tablette ausmacht.

17. Tablette gemäß einem der Ansprüche 15 und 16, wobei das Arginin aus der Gruppe, die aus L-Arginin, L-Arginin-Hydrochlorid, pharmakologisch zulässigen Argininsalzen und Mischungen daraus besteht, ausgewählt wird.

## Revendications

1. Une méthode pour produire des comprimés à libération prolongée comprenant les étapes consistant à :
- mélanger de la L-arginine avec une matrice à libération prolongée ;
- comprimer ledit mélange pour former des comprimés ; et
- recouvrir lesdits comprimés d'un enrobage contenant au moins 10 % en poids d'hydroxypropylméthylcellulose.

2. La méthode de la revendication 1, dans laquelle ladite L-arginine est sélectionnée dans le groupe consistant en hydrochlorure de L-arginine, sels d'arginine acceptables d'un point de vue pharmacologique, et mélanges de ceux-ci.

3. La méthode telle que revendiquée dans l'une ou l'autre des revendications 1 et 2 dans laquelle ladite arginine constitue de 15 % à 60 % en poids du comprimé.

4. La méthode telle que revendiquée dans n'importe quelle revendication précédente, dans laquelle ladite arginine est présente dans une quantité suffisante pour produire des comprimés comprenant ladite L-arginine dans une gamme allant de 150 mg à 2 000 mg.

5. La méthode telle que revendiquée dans n'importe quelle revendication précédente, dans laquelle ladite arginine est présente dans une quantité suffisante pour produire des comprimés ayant 750 mg de L-arginine.

6. La méthode telle que revendiquée dans n'importe quelle revendication précédente, dans laquelle ladite arginine est présente dans une quantité suffisante pour produire des comprimés ayant 350 mg de L-arginine.

7. La méthode telle que revendiquée dans n'importe quelle revendication précédente, dans laquelle ladite L-arginine et ladite matrice à libération prolongée sont mélangées à sec avec un agent de glissement et un agent de remplissage.

8. La méthode de la revendication 7, dans laquelle ledit agent de glissement est sélectionné dans le groupe consistant en silice colloïdale, silice précipitée, et des mélanges de celles-ci.

9. La méthode telle que revendiquée dans n'importe quelle revendication précédente, dans laquelle ladite matrice à libération prolongée est de l'hydroxypropylméthylcellulose (HPMC).

10. La méthode telle que revendiquée dans n'importe quelle revendication précédente, dans laquelle ledit comprimé est recouvert d'un enrobage, ledit enrobage étant un enrobage à base d'éther de cellulose seul ou en combinaison avec de l'éthyl cellulose.

11. La méthode telle que revendiquée dans n'importe quelle revendication précédente, incluant de plus les étapes consistant à mélanger un agent qui renforce la biotransformation de la L-arginine en oxyde nitrique.

12. La méthode de la revendication 11, dans laquelle ledit agent est sélectionné dans le groupe consistant en un agoniste de la NOS, un inhibiteur de la HMG-CoA réductase, et un inhibiteur de l'ACE.

13. Une composition composée d'une quantité efficace d'un point de vue thérapeutique d'arginine ; d'une quantité efficace d'un point de vue thérapeutique d'un inhibiteur de la HMG-CoA réductase autre que la simvastatine ; et d'une matrice polymère à libération prolongée.

14. La composition de la revendication 13, incluant de plus un nitrate.

15. Un comprimé pharmaceutique à libération prolongée composé d'une matrice à libération prolongée ; d'une quantité efficace d'un point de vue thérapeutique d'arginine, et d'un inhibiteur de la HMG-CoA réductase autre que la simvastatine.

16. Le comprimé de la revendication 15 dans lequel ladite arginine représente de 20 % à 60 % en poids dudit comprimé.

17. Le comprimé de l'une ou l'autre des revendications 15 et 16, dans lequel ladite arginine est sélectionnée dans le groupe consistant en L-arginine, hydrochlorure de L-arginine, sels d'arginine acceptables d'un point de vue pharmacologique, et mélanges de ceux-ci.
